# EUROPÄISCHE PATENTSCHRIFT

(11) **EP 2 969 295 B1**
(45) Veröffentlichungstag und Bekanntmachung des Hinweises auf die Patenterteilung: **29.03.2017**
(21) Anmeldenummer: 14706783.9
(22) Anmeldetag: 21.02.2014
(51) Int. Cl.: B21F 45/24

(54) **VORRICHTUNG UND VERFAHREN ZUM HERSTELLEN VON AUS DRAHTABSCHNITTEN GEBOGENEN KLAMMERN**
DEVICE AND METHOD FOR PRODUCING CLAMPS BENT FROM WIRE SECTIONS
DISPOSITIF ET PROCÉDÉ POUR FABRIQUER DES AGRAFES INCURVÉES À PARTIR DE SEGMENTS DE FIL

(30) Priorität: 16.03.2013 DE 102013004652
(43) Veröffentlichungstag der Anmeldung: 20.01.2016
(73) Patentinhaber: Ethicon Endo-Surgery, Inc., Cincinnati, OH 45242 (US)
(72) Erfinder: ALBRECHT, Christoph, 79674 Todtnau-Aftersteg (DE); GOLDMANN, Philipp, 79677 Wembach (DE)
(74) Vertreter: Wegner, Hans
(86) Internationale Anmeldenummer: PCT/EP2014/000462
(87) Internationale Veröffentlichungsnummer: WO 2014/146745

(56) Entgegenhaltungen:
- DE-B1- 1 912 710
- US-A- 362 530
- US-A- 3 403 832
- US-A- 5 060 468
- US-A- 5 381 649

## Beschreibung

Die Erfindung bezieht sich auf eine Vorrichtung zum Herstellen von aus Drahtabschnitten gebogenen Klammern, insbesondere chirurgischen Klammern, mit einer Biege-Einrichtung zum Biegen eines Drahtabschnitts und mit einer Zuführeinrichtung für einen Draht mit einer Schneideeinrichtung zum Abtrennen eines Drahtabschnitts von dem zugeführten Draht.

Solche Vorrichtungen sind in zahlreichen Ausführungen bekannt. Problematisch dabei ist jedoch, dass sich der Drahtabschnitt beim Beaufschlagen durch die Biege-Einrichtung verschieben oder verdrehen kann, so dass die Klammern vergleichsweise großen Produktionstoleranzen unterliegen. Auch beim Abtrennen des Drahtabschnitts von einem längeren Drahtstück können entsprechende Positionsverschiebungen mit der Folge von Maß-Ungenauigkeiten auftreten.

Die US 5 060 468 zeigt und beschreibt eine Vorrichtung nebst Verfahren, bei der ein vorgefertigter Drahtabschnitt in eine Halterung einsetzbar ist. Die freien Enden des Drahtabschnitts werden dann von einer Abschereinrichtung beaufschlagt und dabei umgebogen und gekürzt. Zwar wird der Drahtabschnitt dabei klemmend gehalten, was die Gefahr von Positionsverschiebungen reduziert, jedoch entsteht durch das Kürzen der freien Enden des Drahtabschnitts bei jeder einzelnen Klammer eine große Menge Ausschussmaterial.

Es besteht daher die Aufgabe, eine Vorrichtung der eingangs genannten Art zu schaffen, mit der/dem Klammern mit geringen Fertigungstoleranzen hergestellt werden
können. Zudem soll kein oder nur wenig Ausschussmaterial anfallen.

Die erfindungsgemäße Lösung besteht hinsichtlich der Vorrichtung darin, dass die Schneideeinrichtung zwei Schneide-Stempel zum Schneiden des Drahtes aufweist, und dass eine Haltevorrichtung zum Greifen und Halten des Drahtabschnittes zwischen den beiden Schneide-Stempeln vor dem Abtrennen des Drahtabschnittes und zum Positionieren des abgetrennten Drahtabschnittes an einer Biege-Einrichtung sowie zum Zuführen des zur Klammer gebogenen Drahtabschnitts an eine Vorrichtung zum Einführen der Klammer in eine Kartusche oder einen Klammerspeicher vorgesehen ist.
Da der Draht bereits vor dem Abtrennen im Bereich des abzutrennenden Drahtabschnitts gegriffen wird und während dem Abtrennen und dem Biegen bis zum Zuführen an die Vorrichtung zum Einführen der Klammer in eine Kartusche oder einen Klammerspeicher dauerhaft gehalten wird, ohne den Drahtabschnitt beziehungsweise den zur Klammer gebogenen Drahtabschnitt zwischenzeitlich loszulassen, können die Drahtabschnitte mit einer Wiederholgenauigkeit von 1/100 Millimeter abgeschnitten und gebogen werden. Ein Verrutschen in Längsrichtung oder ein Verdrehen ist sicher vermieden. Zudem wird unnötiger Materialverlust vermieden, da der durch die Schneide-Stempel abgetrennte Drahtabschnitt ohne weitere Kürzungen seiner Länge zur fertigen Klammer umgebogen wird.
Eine bevorzugte Ausführungsform sieht dabei vor, dass die Haltevorrichtung zangenartig ausgebildet ist und einen gegen eine Rückstellkraft aus einer Halteposition in eine Offenstellung bringbaren Haltebereich aufweist. Dies ermöglicht ein einfaches Anbringen der Haltevorrichtung an den Drahtabschnitt und ein versehentliches Lösen ist durch die Rückstellkraft sicher vermieden.

Die Biege-Einrichtung kann insbesondere einen Amboss als Formgebungselement für den Drahtabschnitt aufweisen, der zwischen zwei jeweils gegen eine Rückstellkraft verstellbaren Andrückrollen linearverschiebbar gelagert ist.
Dadurch ist auf einfache Weise ein Umbiegen des Drahtabschnittes zur Klammer möglich. Der Drahtabschnitt wird dabei mit seinem mittleren Bereich, der den Klammerrücken der fertigen Klammer bildet, an eine Stirnseite des Ambosses angelegt. Durch die Beaufschlagung der beidseitig überstehenden freien Enden des Drahtabschnittes werden diese von den Andrückrollen umgebogen und an die Längsseiten des Ambosses angedrückt. Diese umgebogenen Enden des Drahtabschnittes bilden somit die Schenkel der fertigen Klammer. Durch die Formgebung des Ambosses kann die Form der fertigen Klammer festgelegt werden. Beispielsweise können durch eine entsprechende Amboss-Form mit einem sich verjüngenden Bereich die Endbereiche der Schenkel noch zusätzlich nach innen gebogen werden.

Zusätzlich kann die Biege-Einrichtung einen Amboss aufweisen, der Anschlagflächen für die umgebogenen Drahtschenkel und diesen zugeordnete Andrückrollen zum Scheren der Drahtschenkel gegeneinander aufweist.
Nach dem Umbiegen der Drahtschenkel kann die Klammer an einen weiteren Amboss angelegt werden, um die Drahtschenkel aus der flachen Klammer-Ebene auszulenken und gegeneinander zu verschränken. Dies kann insbesondere bei Klammern mit langen Schenkeln sinnvoll sein, damit sich die freien Enden der Schenkel während dem Klammervorgang nicht gegenseitig berühren, was zu einem unvollständigen Klammervorgang und einem unzureichenden Halt führen kann.

Es ist auch möglich, dass der Amboss der Biege-Einrichtung einen kreisabschnittsförmigen Formgebungsbereich für den Drahtabschnitt aufweist, um entsprechend geformte Klammern mit einer Rundung herstellen zu können.

Für eine gleichmäßige und synchrone Bewegung von Haltevorrichtung und Amboss ist es zweckmäßig, wenn die Haltevorrichtung mit dem Amboss der Biege-Einrichtung lösbar verbindbar ist.

Die Zuführeinrichtung kann insbesondere eine Drahtrolle als Vorrat für den zuzuführenden Draht aufweisen. Dies ermöglicht es, auf geringem Raum eine große Menge Draht bereitzustellen.

Um Krümmungen des von der Drahtrolle abgewickelten Drahtes zu beseitigen und sicherzustellen, dass der Draht im Bereich der Schneide-Stempel in gerader Form zugeführt wird, kann die Zuführeinrichtung einen Richtapparat mit mehreren, beidseits des Drahtes angeordneten Führungsrollen zur Ausrichtung des von der Drahtrolle abgewickelten Drahtes aufweisen.

Ebenfalls zum Richten des abgewickelten Drahtes kann die Zuführeinrichtung zwei in Draht-Vorschubrichtung linearverschiebbare Klemmelemente aufweisen. Ein Abschnitt des Drahtes wird von den beiden zueinander beabstandeten Klemmelementen klemmend gehalten. Eines der Klemmelemente wird dann durch eine Linearverschiebung um eine geringe Strecke von dem anderen Klemmelement wegbewegt, so dass sich der Abstand der beiden Klemmelemente vergrößert. Gegebenenfalls können auch beide Klemmelemente in entgegengesetzte Richtungen bewegt werden. Dadurch wird der zwischen den Klemmelementen eingeklemmte Drahtabschnitt gereckt und eventuelle Krümmungen beseitigt.

Die Schneide-Stempel können vorzugsweise jeweils eine schräge Schnittkante aufweisen. Dadurch kann der Drahtabschnitt so abgeschnitten werden, dass die freien Enden des Drahtabschnitts eine entsprechende Einführschräge aufweisen. Zusätzliche. Schneidvorgänge an den Enden des Drahtabschnitts sind somit nicht erforderlich.

Um auf einfache Art die Orientierung der Schnittkante variieren zu können, ist es vorteilhaft, wenn die Schneide-Stempel jeweils in einer Stempel-Führung gelagert sind, die um eine in Draht-Längsrichtung orientierte Achse verschwenkbar und/oder bezüglich der Draht-Längsrichtung um 180° gedreht anordenbar ist.

Der Biege-Einrichtung kann zumindest eine Bearbeitungseinrichtung für den Draht oder den Drahtabschnitt vor- und/oder nachgeordnet sein. Beispielsweise können vor dem Biegen Materialschwächungen in den Drahtabschnitt eingebracht werden, um so an diesen Stellen ein einfacheres Biegen zu ermöglichen. Bei entsprechender Anforderung an die fertige Klammer können beispielsweise auch die freien Enden des Drahtabschnitts bearbeitet, beispielsweise geschliffen, gespitzt oder verdreht werden. Die Bearbeitungseinrichtung kann auch zum Anbringen einer Markierung, beispielsweise eines Barcodes, eines Datumcodes oder eines Identitätscodes ausgebildet sein. Die Markierung kann dabei beispielsweise über eine Lasergravur erfolgen.

Es kann zweckmäßig sein, wenn eine Kontrollvorrichtung für den gebogenen Drahtabschnitt vorgesehen ist. Dabei können insbesondere die Form der gebogenen Klammer und/oder die Federkraft der Klammer-Schenkel überprüft werden.

Eine bevorzugte Ausführungsform sieht vor, dass die Vorrichtung zum Einführen der Klammer in eine Kartusche oder einen Klammerspeicher einen Schieber zur Beaufschlagung der Klammer an deren Klammerrücken aufweist. Da somit die freien Enden der Klammer-Schenkel, die den empfindlichsten Teil der Klammer darstellen, nicht beaufschlagt werden, sind Beschädigungen an diesen während dem Einführen der Klammer sicher vermieden.

Dabei ist es zweckmäßig, wenn der Schieber dem Drahtabschnitt im Beaufschlagungsbereich formangepasst ist. Dies ermöglicht eine sichere Führung der Klammer durch den Schieber.

Es kann zudem zweckmäßig sein, wenn der Schieber im Beaufschlagungsbereich dünner ist als der zu beaufschlagende Drahtabschnitt. Beispielsweise kann der Schieber um 0,01mm bis 0,1mm dünner sein als der Drahtabschnitt. Dadurch kann der Schieber die Klammer in eine Kartusche oder einen Klammerspeicher einschieben, ohne selbst an der Kartusche oder dem Klammerspeicher anzustoßen.

Bevorzugt ist eine Antriebseinheit für eine Linearverschiebung des Schiebers vorgesehen.

Die Antriebseinheit kann einen Kraftsensor sowie eine Steuereinheit zum Steuern des Schiebers in Abhängigkeit der Sensor-Signale aufweisen. Eine zu starke Kraftbeaufschlagung an der Antriebseinheit kann beispielsweise auf eine fehlerhaft positionierte Klammer hindeuten, die nicht korrekt in die Kartusche oder einen Klammerspeicher eingeführt werden kann. In einem solchen Fall kann die Antriebseinheit von der Steuereinheit gestoppt werden, um Beschädigungen an der Vorrichtung zu vermeiden.

Es ist auch möglich, dass die Antriebseinheit eine kraftgesteuerte Kupplungseinheit aufweist. Bei einer zu hohen Kraftbeaufschlagung kann die Kupplungseinheit auskuppeln, so dass der Schieber nicht mehr angetrieben wird. Die Kupplungseinheit kann beispielsweise durch eine Federklemmung, die bei einer zu hohen Last durchrutscht, gebildet sein.

Die Vorrichtung zum Einführen der Klammer in eine Kartusche oder einen Klammerspeicher kann bevorzugt ein Gehäuse mit einer Führung für den Schieber sowie einer Einlegeöffnung zum Einlegen jeweils einer Klammer aufweisen.

Um nacheinander die einzelnen Klammer-Aufnahmen eines Klammerspeichers oder einer Kartusche mit der Vorrichtung zum Einführen der Klammer befüllen zu können, ist es zweckmäßig, wenn eine Positioniervorrichtung zum Positionieren eines Klammerspeichers oder einer Kartusche vor dem abgabeseitigen Ende der Vorrichtung zum Einführen der Klammer in eine Kartusche oder einen Klammerspeicher vorgesehen ist. Somit kann jeweils eine Klammer-Aufnahme vor der ortsfesten Vorrichtung positioniert werden, um jeweils eine Klammer mit dem Schieber in die Klammer-Aufnahme einzuführen.

Die einzelnen Komponenten der Vorrichtung (Zuführeinrichtung, Schneideeinrichtung, Haltevorrichtung, Biege-Einrichtung, Vorrichtung zum Einführen der Klammer in eine Kartusche oder einen Klammerspeicher) können zueinander beabstandet an einzelnen Bearbeitungsstationen angeordnet sein.
Bevorzugt sind die einzelnen Komponenten der Vorrichtung jedoch an einer gemeinsamen Bearbeitungsstation angeordnet. Dies ermöglicht einen kompakten, platzsparenden Aufbau der gesamten Vorrichtung.

Hinsichtlich des Verfahrens ist die Erfindung dadurch gekennzeichnet, dass der Drahtabschnitt von einem Draht abgeschnitten und die freien Enden des Drahtabschnittes zu Drahtschenkeln umgebogen werden, dass der zur Klammer gebogene Drahtabschnitt in eine Kartusche oder einen Klammerspeicher eingeführt wird, und dass der Drahtabschnitt vor dem Abschneiden gegriffen und während dem Abschneiden, dem Umbiegen, und bis zum Zuführen des zur Klammer gebogenen Drahtabschnitts an eine Vorrichtung zum Einführen der Klammer in eine Kartusche oder einen Klammerspeicher durchgängig gehalten wird.
Dabei ergeben sich die bereits bei der Beschreibung der erfindungsgemäßen Vorrichtung erläuterten Vorteile.
Vorzugsweise wird der Draht von einer Drahtrolle abgewickelt.
Dabei ist vorteilhaft, wenn der Draht vor dem Abschneiden ausgerichtet und/oder gereckt wird. Dadurch werden eventuelle Biegungen beseitigt und der.Draht ist vor dem Abschneiden und Biegen zur Klammer präzise gerade ausgerichtet, wodurch die gewünschte Präzision der Klammern erreicht werden kann.
Das Biegen des Drahtabschnittes zur Klammer kann besonders gut und präzise erfolgen, wenn der abgeschnittene Drahtabschnitt mit seinem den Klammerrücken der fertigen Klammer bildenden mittleren Bereich an einen Amboss angelegt und der Amboss zwischen zwei druckbeaufschlagten Andrückrollen hindurchbewegt wird und dadurch die über den Amboss überstehenden Drahtbereiche umgebogen werden.

Es kann zweckmäßig sein, wenn die umgebogenen Drahtschenkel aus der Klammer-Ebene heraus umgebogen und gegeneinander geschert werden. Insbesondere bei Klammern mit langen Schenkeln ist so sicher vermieden, dass sich beim Klammern die Drahtschenkel an ihren Enden berühren und so ein sicheres Klammern beeinträchtigt werden könnte.

Der Drahtabschnitt kann vor und/oder nach dem Biegen bearbeitet werden. Beispielsweise können Materialschwächungen in den Drahtabschnitt eingebracht oder die Draht-Enden geschliffen oder angespitzt werden. Es ist auch möglich, Markierungen an den Drahtabschnitt anzubringen.

Nachstehend ist die Erfindung anhand der Zeichnungen näher erläutert.

Es zeigt, zum Teil schematisiert:
- Fig. 1: eine Zuführeinrichtung mit einem Richtapparat und Klemmelementen sowie eine Schneideeinrichtung,
- Fig. 2: einen Schneide-Stempel und eine Stempel-Führung,
- Fig. 3: Drahthaltevorrichtungen mit jeweils einem daran gehaltenen Drahtabschnitt,
- Fig. 4: eine Bearbeitungseinrichtung zum Bearbeiten eines Drahtabschnitts,
- Fig. 5: eine Drahthaltevorrichtung mit einem an der Bearbeitungseinrichtung aus Figur 4 bearbeiteten Drahtabschnitt,
- Fig. 6: eine perspektivische Ansicht einer Biege-Einrichtung zum Biegen eines Drahtabschnitts,
- Fig. 7: eine Aufsicht einer Biege-Einrichtung zum Biegen eines Drahtabschnitts,
- Fig. 8: einen ungebogenen Drahtabschnitt,
- Fig. 9: eine Detaildarstellung des gemäß Figur 7 umgebogenen Drahtabschnitts,
- Fig. 10 und Fig. 11: eine Biege-Einric'htung zum Scheren der umgebogenen Draht-Schenkel,
- Fig. 12: Seitenansichten einer Klammer vor und nach dem Scheren der Draht-Schenkel,
- Fig. 13 bis Fig. 17: jeweils eine Vorrichtung zum Einführen einer Klammer in eine Kartusche oder einen Klammerspeicher,
- Fig. 18: eine Vorrichtung zum Einführen einer Klammer in eine Kartusche mit einer davor positionierbaren Kartusche,
- Fig. 19: eine mit Klammern befüllte Kartusche,
- Fig. 20: einen mit Klammern befüllten Klammerspeicher,
- Fig. 21: einen Klammerspeicher als Vorrat zum Zuführen von Klammern an eine Vorrichtung zum Einführen einer Klammer in eine Kartusche und
- Fig. 22: eine schematische Darstellung der einzelnen Bearbeitungsstationen der Vorrichtung.

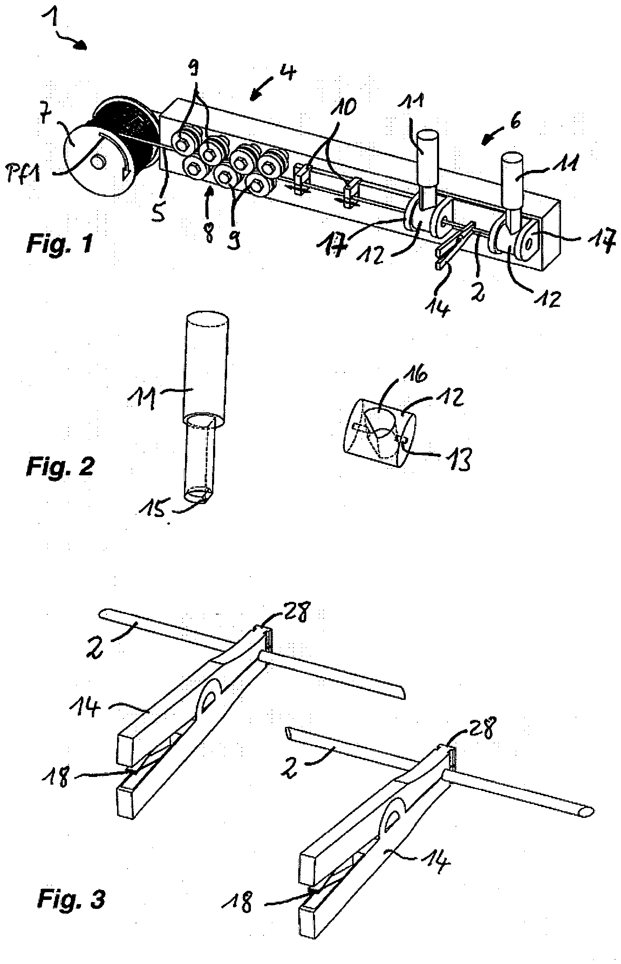

Eine im Ganzen mit 1 bezeichnete Vorrichtung zum Herstellen von aus Drahtabschnitten 2 gebogenen Klammern 3 weist gemäß Figur 1 eine Zuführeinrichtung 4 zum Zuführen von Draht 5 an eine Schneideeinrichtung 6 auf. Als Vorrat für den zuzuführenden Draht 5 ist eine Drahtrolle 7 vorgesehen, von der der Draht 5 durch Drehung der Drahtrolle 7 in Richtung des Pfeils Pf1 abgewickelt wird. Der Draht kann insbesondere aus Titan oder einer Titan-Aluminium-Vanadium-Legierung bestehen.

Um Krümmungen des abgewickelten Drahtes 5 zu beseitigen weist die Zuführeinrichtung 4 einen Richtapparat 8 mit mehreren, beidseits des Drahtes 5 angeordneten Führungsrollen 9 auf. Eventuelle Krümmungen des Drahtes 5 werden beim Durchlaufen des Drahtes 5 durch die abwechselnd beidseits des Drahtes 5 angeordneten Führungsrollen 9 beseitigt.

Nach dem Durchlaufen des Richtapparates 8 wird der weiterbeförderte Draht 5 von zwei Klemmelementen 10 gegriffen. Das in Figur 1 linke Klemmelement 10 hält den Draht 5 dabei fest, während das rechte Klemmelement 10 um eine geringe Strecke nach rechts, von der Drahtrolle 7 weg, bewegt wird. Dadurch wird der Draht 5 im Bereich zwischen den Klemmelementen 10 gereckt und eventuelle verbliebene Biegungen dadurch beseitigt.

Beide Klemmelemente 10 werden anschließend synchron nach rechts bewegt, so dass ein zuvor gereckter Abschnitt des Drahtes 5 rechts von den Klemmelementen 10 in den Bereich der Schneideeinrichtung 6 geschoben wird. Die Klemmelemente 10 werden danach geöffnet und in ihre Ausgangspositionen zurückbewegt, so dass sie einen weiteren Bereich des Drahtes 5, der zuvor die Führungsrollen 9 durchlaufen hat, klemmend halten können.

Die Schneideeinrichtung 6 weist zwei Schneide-Stempel 11 auf, die jeweils in einer Stempel-Führung 12 gelagert sind, wobei der Draht 5 jeweils durch eine Durchgangsöffnung 13 (Figur 2) der Stempel-Führungen 12 hindurch geführt ist.
Nach dem Zuführen des Drahtes 5 an die Schneideeinrichtung 6 wird der Draht 5 zwischen den beiden Schneide-Stempeln 11 mit einer Haltevorrichtung 14 gegriffen. Erst danach wird der Abschnitt des Drahtes 5 zwischen den beiden Schneide-Stempeln 11 durch Betätigung der Schneide-Stempel 11 von dem zugeführten Draht 5 abgeschnitten. Durch die Form der Schneidkante 15 der Schneide-Stempel 11 (Figur 2) wird der Draht 5 von dem in Figur 1 linken Schneide-Stempel 11 zweifach geschnitten. Wird der Draht 5 nur soweit vorgeschoben, dass sein freies Ende innerhalb der Aufnahmeöffnung 16 für den Schneide-Stempel 11 der rechten Stempel-Führung 12 angeordnet ist, so wird der Draht 5 vom rechten Schneide-Stempel 11 gemäß Figur 1 nur an einer Stelle geschnitten. Dadurch wird Materialverlust durch Schnittabfall vermieden. Wird der Draht 5 jedoch weiter in die rechte Stempel-Führung 12 eingeschoben, so wird der Draht 5 auch vom rechten Schneide-Stempel 11 an zwei Positionen geschnitten. Dies hat jedoch keine negativen Auswirkungen auf die Beschaffenheit des abgeschnittenen Drahtabschnitts 2 (Figur 3).

Der Draht 5 kann innerhalb der Zuführeinrichtung 4 durch zusätzliche, nicht dargestellte Führungselemente geführt sein.

Die Position der Stempel-Führungen 12 und somit der Schneide-Stempel 11 kann in Längsrichtung des Drahtes 5 verstellt werden, um einerseits unterschiedlich lange Drahtabschnitte 2 abschneiden zu können und um andererseits ein ungehindertes Entnehmen eines abgeschnittenen Drahtabschnitts 2 aus dem Bereich zwischen den beiden Stempel-Führungen 12 zu ermöglichen.

In Figur 2 ist einerseits ein Schneide-Stempel 11 dargestellt, wobei dessen keilförmige Schneidekante 15 erkennbar ist. Andererseits ist eine Stempel-Führung 12 dargestellt mit der Aufnahmeöffnung 16 für den Schneide-Stempel 11 und der Durchgangsöffnung 13 für den Draht 5.

Die Stempel-Führungen 12 sind jeweils in Halterungen 17 (Figur 1) lösbar gehalten. Durch seitenvertauschtes Einsetzen der Stempel-Führung 12 in die Halterung 17 kann die Orientierung der Schnittkante des abgeschnittenen Drahtabschnitts 2 variiert werden, wie dies anhand der beiden in Figur 3 dargestellten Drahtabschnitte 2 verdeutlich ist.
Wenn andere Schnittwinkel oder andere Schnittformen gewünscht sind, können die Stempel-Führungen 12 durch entsprechend ausgestaltete Exemplare ausgetauscht werden.

In Figur 3 ist auch gut zu erkennen, dass die Haltevorrichtung 14 zangenartig ausgebildet ist und der den Drahtabschnitt 2 haltende Haltebereich gegen die Rückstellkraft einer Spannfeder 18 aus der dargestellten Halteposition in eine Offenstellung bringbar ist.

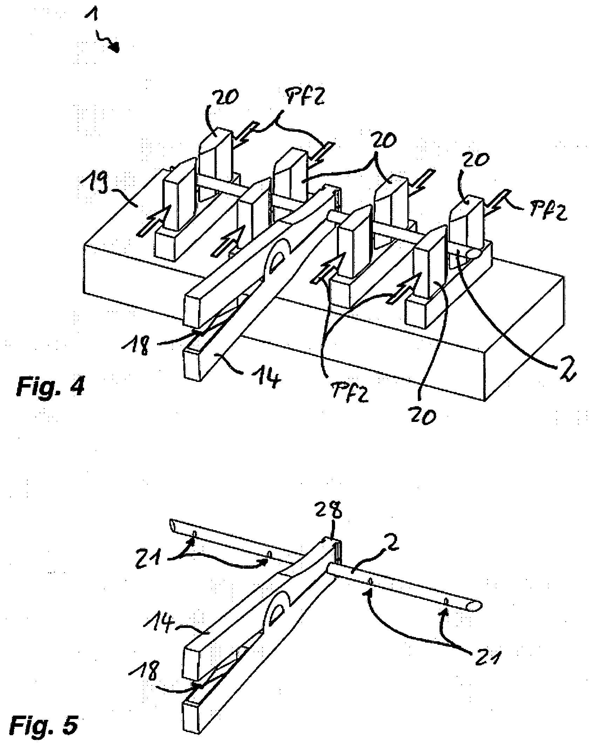

Nach dem Abtrennen des Drahtabschnitts 2 wird dieser mit der Haltevorrichtung 14 einer Bearbeitungseinrichtung 19 (Figur 4), die ebenfalls Bestandteil der gesamten Vorrichtung 1 ist, zugeführt. An der Bearbeitungsstation 19 werden mit Beaufschlagungskeilen 20 Quetschungen oder Materialschwächungen 21 in den Drahtabschnitt 2 (Figur 5) eingebracht. Dazu werden die Beaufschlagungskeile 20 gemäß den Pfeilen Pf2 aus Figur 4 mit einer entsprechenden Andruckskraft an den Drahtabschnitt 2 heranbewegt. Durch die Materialschwächungen 21 kann der Drahtabschnitt 2 an diesen Stellen besser gebogen werden.
Die Bearbeitungseinrichtung 19 kann auch weitere, nicht dargestellte Bearbeitungswerkzeuge aufweisen, beispielsweise zum Schleifen oder Anspitzen der freien Drahtenden. Diese verschiedenen Bearbeitungen können an einer einzigen Bearbeitungseinrichtung 19 durchgeführt werden, oder es können mehrere Bearbeitungseinrichtungen 19 vorgesehen sein, an denen der Drahtabschnitt 2 nacheinander positioniert wird.

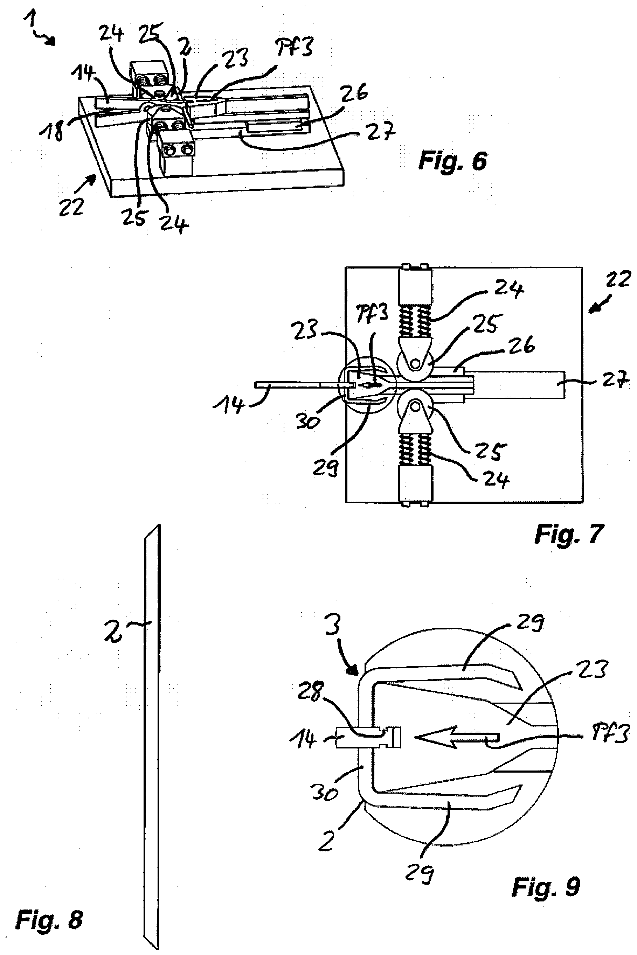

Anschließend wird der Drahtabschnitt 2 gemäß Figuren 6, 7 und 9 einer Biege-Einrichtung 22 zugeführt. Die Biege-Einrichtung 22 weist einen Amboss 23 als Formgebungselement für den Drahtabschnitt 2 auf. Der Amboss 23 ist zwischen zwei jeweils gegen die Rückstellkraft von Druckfedern 24 verstellbaren Andrückrollen 25 linearverschiebbar gelagert. Der Amboss 23 ist dazu gemäß Figur 6 mit einem Führungswagen 26 auf einer Führungsschiene 27 gelagert. Damit der Drahtabschnitt 2 auch während dem Biegevorgang von der Haltevorrichtung 14 gehalten wird und sich die Haltevorrichtung 14 und der Amboss 23 synchron bewegen, sind die Haltevorrichtung 14 und der Amboss 23 lösbar miteinander verbindbar. Die Haltevorrichtung 14 weist dazu ein profiliertes Verbindungsende 28 auf (Figur 9), das in eine entsprechend gegenprofilierte Ausnehmung im Amboss 23 einsetzbar ist.
Wie in Figur 6 gezeigt, wird die Haltevorrichtung 14 mit dem Drahtabschnitt 2 an den vor den Andrückrollen 25 positionierten Amboss 23 angedockt. Gemäß dem Pfeil Pf3 wird der Amboss 23 mit der Haltevorrichtung 14 zwischen den Andrückrollen 25 hindurchgezogen. Dadurch werden die über die Stirnseite des Amboss' 23, an der der Drahtabschnitt 2 anliegt, überstehenden Enden des Drahtabschnitts 2 von den Andrückrollen 25 umgebogen und an die Seitenwandungen des Amboss' 23 angedrückt. Durch die Form des Amboss' 23 und die Materialeigenschaften des Drahtabschnitts 2 federn die umgebogenen Drahtschenkel 29 nach dem Durchziehen durch die Andrückrollen 25 wieder etwas zurück, was in Figur 7 und insbesondere in der Detaildarstellung gemäß Figur 9 deutlich zu erkennen ist. Der an der Stirnseite des Amboss' 23 anliegende Bereich des Drahtabschnitts 2 bildet den Klammerrücken 30 der fertigen Klammer 3

Figur 8 zeigt den ungebogenen Drahtabschnitt 2 vor dem Biegen.

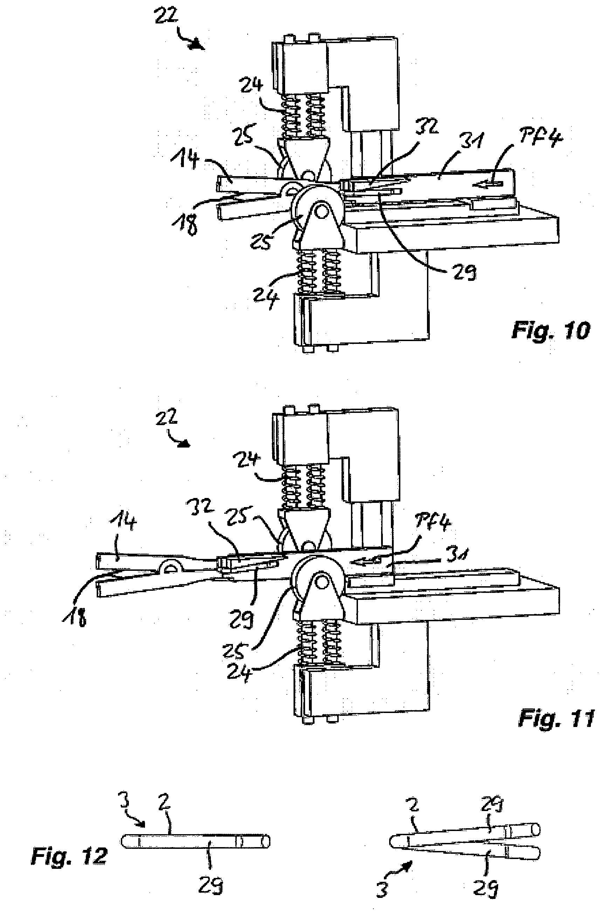

Die Biege-Einrichtung 22 kann einen weiteren Amboss 31 gemäß Figuren 10 und 11 aufweisen, der Anschlagflächen 32 für die umgebogenen Drahtschenkel 29 und diesen zugeordnete Andrückrollen 25 zum Scheren der Drahtschenkel 29 gegeneinander aufweist. Der Drahtabschnitt 2 mit den umgebogenen Drahtschenkeln 29 wird, weiterhin von der Haltevorrichtung 14 gehalten, an den weiteren Amboss 31 angedockt, und dieser wird gemäß Pfeil Pf4 an den mit Druckfedern 24 gelagerten Andrückrollen 25 vorbei bewegt. Durch die Beaufschlagung der Drahtschenkel 29 durch die Andrückrollen 25 und die Ausgestaltung der als Begrenzung wirkenden Anschlagflächen 32 des Amboss' 31 werden die Drahtschenkel 29 in gegensätzlichen Richtungen aus ihrer ursprünglichen Ebene ausgelenkt. Auch nach diesem Biegevorgang federn die Drahtschenkel 29 elastisch zurück.

In Figur 12 ist der zur Klammer 3 gebogene Drahtabschnitt 2 vor (links) und nach (rechts) dem Biegen an der Einrichtung gemäß Figuren 10 und 11 in Seitenansicht dargestellt. Das Verschränken der Drahtschenkel 29 verhindert, dass sich die freien Enden der Klammer 3 beim Klammervorgang treffen können.

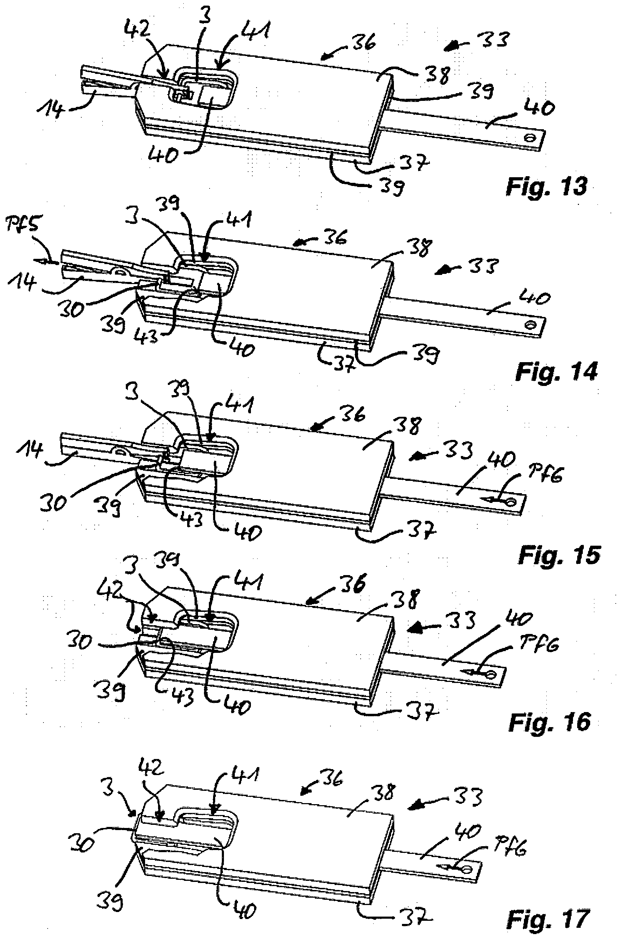

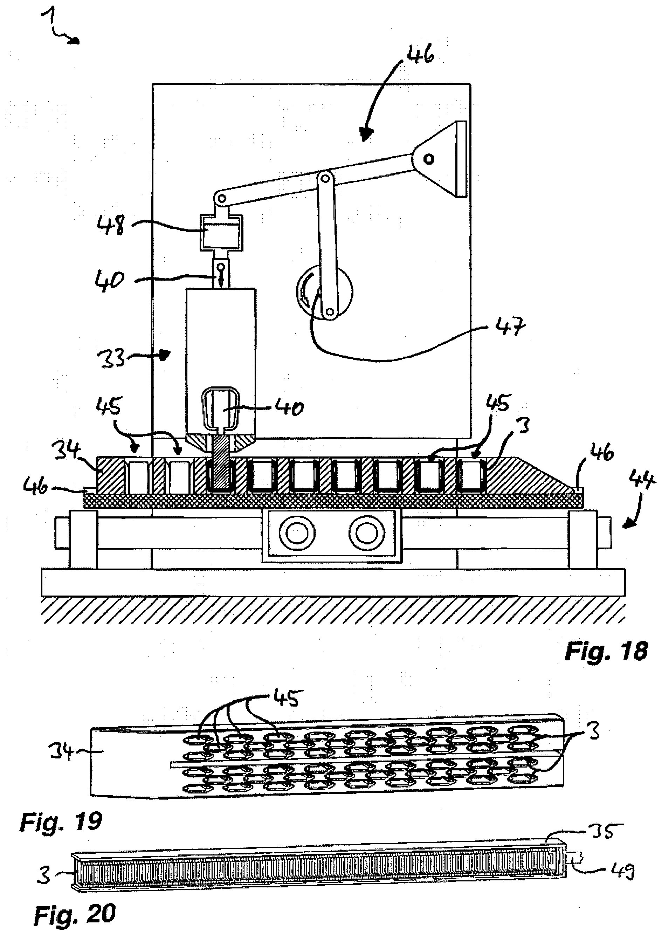

Die Figuren 13 bis 17 zeigen jeweils eine Vorrichtung 33 zum Einführen einer Klammer 3 in eine Kartusche 34 (Figur 19) oder einen Klammerspeicher 35 (Figur 20). Die Vorrichtung 33 weist dabei ein Gehäuse 36 mit einer Grundplatte 37, einer Deckplatte 38 sowie dazwischen liegenden Zwischenplatten 39 auf. Die Zwischenplatten 39 bilden seitliche Führungen für einen Schieber 40, der längsverschiebbar in dem Gehäuse 36 gelagert ist. Über eine Einlegeöffnung 41 wird eine Klammer 3 in die Vorrichtung 33 eingelegt (Figur 13). Die Klammer 3 wird dabei immer noch von der Haltevorrichtung 14 gehalten. Die Grundplatte 37 und die Deckplatte 38 weisen am stirnseitigen Ende des Gehäuses 33 jeweils eine Ausnehmung 42 zur Aufnahme der Haltevorrichtung 14 auf. Nach dem Einführen der Klammer 3 mit der Haltevorrichtung 14 durch die Einlegeöffnung 41 in die Vorrichtung 33 liegt die Klammer 3 auf der Grundplatte 37 auf und ist seitlich durch die Zwischenplatten 39 geführt. Durch Bewegung der Haltevorrichtung 14 in Richtung des Pfeils Pf5 (Figur 14) wird die Klammer 3 mit ihrem Klammerrücken 30 unter die Deckplatte 38 gezogen, so dass die Klammer 3 auch zwischen Grundplatte 37 und Deckplatte 38 festgelegt ist.

In den Figuren 14 bis 17 ist zur besseren Übersicht die Deckplatte 38 teilweise gebrochen dargestellt.

Gemäß Figur 15 wird die Haltevorrichtung 14 in ihre Offenstellung gebracht und von der Klammer 3 entfernt. Der Schieber 40 wird gemäß Pfeil Pf6 in Richtung der Klammer 3 verschoben, bis der Schieber 40 den Klammerrücken 30 der Klammer 3 beaufschlagt (Figur 16). Das Beaufschlagungsende 43 des Schiebers 40 ist dabei dem Klammerrücken 30 formangepasst, so dass die Klammer 3 sicher und ohne der Gefahr einer Beschädigung von dem Schieber 40 beaufschlagt und verschoben werden kann.
Durch weiteres Linearverschieben des Schiebers 40 wird die Klammer 3 aus dem Gehäuse 36 und der Vorrichtung 33 herausgeschoben (Figur 17) und in eine Kartusche gemäß Figur 19 oder einen Klammerspeicher gemäß Figur 20 eingeschoben.

Denkbar wären auch Ausführungen, bei denen eine Klammer 3 so von der Haltevorrichtung 14 gehalten wird, dass die Haltevorrichtung 14 gegenüber der gezeigten Darstellung um 90° nach oben verschwenkt ist. Dann könnten die Grundplatte 37 und die Deckplatte 38 ohne Ausnehmungen 42 ausgebildet sein.
Denkbar wäre auch ein Einführen der Klammer 3 von der in Figuren 13 bis 17 linken Seite des Gehäuses 36, durch die die Klammer 3 später auch wieder mit dem Schieber 40 aus dem Gehäuse 36 ausgeschoben wird. Bei einer solchen Ausführungsform wäre die Einlegeöffnung 41 in der Deckplatte 38 nicht erforderlich. Dabei ist jedoch auf eine präzise Führung der Klammer 3 zu achten, um die dann beim Einführen der Klammer 3 in das Gehäuse 36 in Vorschubrichtung vorne liegenden freien Enden der Klammer 3 nicht zu beschädigen.

Das Einschieben einer Klammer 3 in eine Kartusche 34 ist auch in Figur 18 dargestellt. Unterhalb der Vorrichtung 33 zum Einführen der Klammer 3 ist eine Kartusche 34 mit mehreren Klammer-Aufnahmen 45 zur Aufnahme der Klammern 3 angeordnet. Mittels einer Positioniervorrichtung 44 ist jeweils eine der Klammer-Aufnahmen 45 vor der Vorrichtung 33 positionierbar. Die Kartusche 34, die über eine Spannvorrichtung 46 lösbar an der Positioniervorrichtung 44 fixiert ist, ist mittels der als Mehrachs-System ausgebildeten Positioniervorrichtung 44 innerhalb einer Ebene positionierbar. Somit kann jeweils eine Klammer-Aufnahme 45 vor die Vorrichtung 33 gebracht werden, um nacheinander alle Klammer-Aufnahmen 45 der Kartusche 34 mit jeweils einer Klammer 3 zu befüllen (Figur 19).

Gemäß Figur 18 ist eine Antriebseinheit 46 vorgesehen, die die Drehbewegung eines Antriebsmotors 47 in eine Linearbewegung zum verschieben des Schiebers 40 umsetzt. Die Antriebseinheit 46 weist im Bereich des Schiebers 40 einen Kraftsensor 48 auf. Bei einer fehlerhaften Positionierung der Kartusche 34 oder einer defekten oder falsch positionierten Klammer 3 treten erhöhte Kräfte an dem Kraftsensor 48 auf. Über eine nicht dargestellte Steuereinheit kann dann die Antriebseinheit 46 gestoppt werden, um weitere Beschädigungen zu vermeiden.

Figur 19 zeigt eine mit Klammern 3 befüllte Kartusche 34, wie sie beispielsweise im medizinischen Bereich zum Klammern von Wunden eingesetzt werden kann.

Alternativ können die Klammern 3 auch in einen Klammerspeicher 35 gemäß Figur 20 eingesetzt werden. In einem solchen Klammerspeicher 35 können die Klammern 3 für eine spätere Verwendung bevorratet werden.

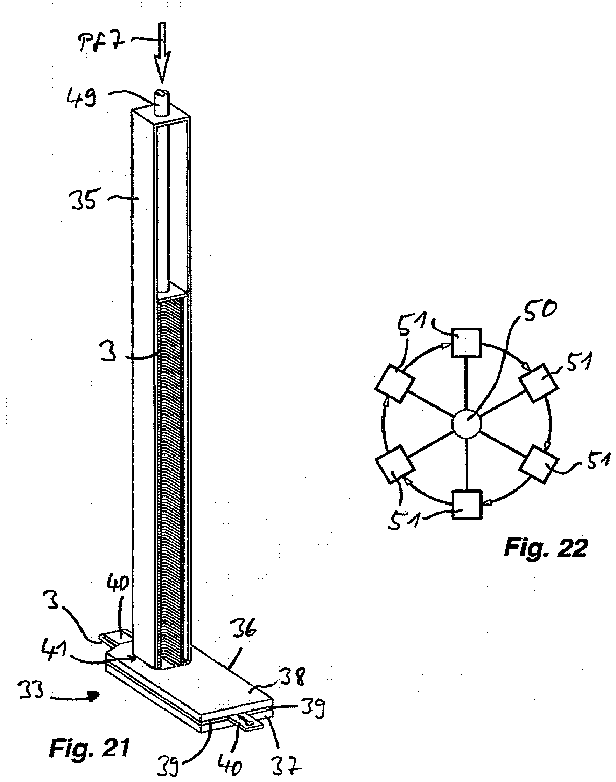

Beispielsweise kann ein Klammerspeicher 35 wie in Figur 21 dargestellt selbst als Zuführ-Vorrat für eine Vorrichtung 33 zum Einführen einer Klammer 3 in eine Kartusche 34 verwendet werden. Ein druckbeaufschlagter (Pfeil Pf7) Schiebestift 49 schiebt die im Klammerspeicher 35 befindlichen Klammern 3 nach unten und führt jeweils eine Klammer 3 in die Einlegeöffnung 41 des Gehäuses 36, wo die Klammer 3 von dem Schieber 40 erfasst und in eine in Figur 21 nicht dargestellte Kartusche eingesetzt wird.

Figur 22 stellt schematisch den Bearbeitungs-Ablauf mit den einzelnen, zuvor beschriebenen Bearbeitungsschritten und den einzelnen Bearbeitungsstationen dar. Eine zentrale Steuerung 50 steuert dabei die Abläufe an den einzelnen Bearbeitungsstationen 51. Die einzelnen Bearbeitungsstationen 51, beispielsweise zum Abschneiden des Drahtabschnitts oder zum Biegen des Drahtabschnitts, müssen dabei nicht wie durch Figur 22 symbolisiert räumlich getrennt voneinander liegend, sondern können auch in einer gemeinsamen, räumlich kompakten Verarbeitungsstation zusammengefasst sein.
Einzelne Bearbeitungsschritte können auch zeitgleich erfolgen, beispielsweise kann eine Klammer 3 in eine Kartusche 34 eingeschoben werden, während ein neuer Drahtabschnitt 2 abgeschnitten und umgebogen wird.
Denkbar sind auch Zwillingsmaschinen, bei denen einzelne Bearbeitungsschritte an mehrfach vorhandenen Vorrichtungen parallel erfolgen.

Nachfolgend werden bevorzugte Ausführungsformen beschrieben, um ein tieferes Verständnis der Erfindung zu ermöglichen:
1. Vorrichtung (1) zum Herstellen von aus Drahtabschnitten (2) gebogenen Klammern (3), insbesondere chirurgischen Klammern, mit einer Biege-Einrichtung (22) zum Biegen eines Drahtabschnitts (2) und mit einer Zuführeinrichtung (4) für einen Draht (5) mit einer Schneideeinrichtung (6) zum Abtrennen eines Drahtabschnitts (2) von dem zugeführten Draht (5), dadurch gekennzeichnet, dass die Schneideeinrichtung (6) zwei Schneide-Stempel (11) zum Schneiden des Drahtes (5) aufweist, und dass eine Haltevorrichtung (14) zum Greifen und Halten des Drahtabschnittes (2) zwischen den beiden Schneide-Stempeln (11) vor dem Abtrennen des Drahtabschnittes (2) und zum Positionieren des abgetrennten Drahtabschnittes (2) an einer Biege-Einrichtung (22) sowie zum Zuführen des zur Klammer (3) gebogenen Drahtabschnitts (2) an eine Vorrichtung (33) zum Einführen der Klammer (3) in eine Kartusche (34) oder einen Klammerspeicher (35) vorgesehen ist.
2. Vorrichtung nach Ausführungsform 1, dadurch gekennzeichnet, dass die Haltevorrichtung (14) zangenartig ausgebildet ist und einen gegen eine Rückstellkraft aus einer Halteposition in eine Offenstellung bringbaren Haltebereich aufweist.
3. Vorrichtung nach Ausführungsform 1 oder 2, dadurch gekennzeichnet, dass die Biege-Einrichtung (22) einen Amboss (23) als Formgebungselement für den Drahtabschnitt (2) aufweist, der zwischen zwei jeweils gegen eine Rückstellkraft verstellbaren Andrückrollen (25) linearverschiebbar gelagert ist.
4. Vorrichtung nach einer der Ausführungsformen 1 bis 3, dadurch gekennzeichnet, dass die Biege-Einrichtung (22) einen Amboss (31) aufweist, der Anschlagflächen (32) für die umgebogenen Drahtschenkel (29) und diesen zugeordnete Andrückrollen (25) zum Scheren der Drahtschenkel (29) gegeneinander aufweist.
5. Vorrichtung nach Ausführungsform 3, dadurch gekennzeichnet, dass der Amboss (23) der Biege-Einrichtung (22) einen kreisabschnittsförmigen Formgebungsbereich für den Drahtabschnitt (2) aufweist.
6. Vorrichtung nach einer der Ausführungsformen 3 bis 5, dadurch gekennzeichnet, dass die Haltevorrichtung (14) mit dem Amboss (23, 31) der Biege-Einrichtung (22) lösbar verbindbar ist.
7. Vorrichtung nach einer der Ausführungsformen 1 bis 6, dadurch gekennzeichnet, dass die Zuführeinrichtung (4) eine Drahtrolle (7) als Vorrat für den zuzuführenden Draht (5) aufweist.
8. Vorrichtung nach einer der Ausführungsformen 1 bis 7, dadurch gekennzeichnet, dass die Zuführeinrichtung (4) einen Richtapparat (8) mit mehreren, beidseits des Drahtes (5) angeordneten Führungsrollen (9) zur Ausrichtung des von der Drahtrolle (7) abgewickelten Drahtes (5) aufweist.
9. Vorrichtung nach einer der Ausführungsformen 1 bis 8, dadurch gekennzeichnet, dass die Zuführeinrichtung (4) zwei in Draht-Vorschubrichtung linearverschiebbare Klemmelemente (10) aufweist.
10. Vorrichtung nach einer der Ausführungsformen 1 bis 9, dadurch gekennzeichnet, dass die Schneide-Stempel (11) jeweils eine schräge Schneidkante (15) aufweisen.
11. Vorrichtung nach einer der Ausführungsformen 1 bis 10, dadurch gekennzeichnet, dass die Schneide-Stempel (11) jeweils in einer Stempel-Führung (12) gelagert sind, die um eine in Draht-Längsrichtung orientierte Achse verschwenkbar und/oder bezüglich der Draht-Längsrichtung um 180° gedreht anordenbar ist.
12. Vorrichtung nach einer der Ausführungsformen 1 bis 11, dadurch gekennzeichnet, dass der Biege-Einrichtung (22) zumindest eine Bearbeitungseinrichtung (19) für den Draht (5) oder den Drahtabschnitt (2) vor- und/oder nachgeordnet ist.
13. Vorrichtung nach einer der Ausführungsformen 1 bis 12, dadurch gekennzeichnet, dass eine Kontrollvorrichtung für den gebogenen Drahtabschnitt (2) vorgesehen ist.
14. Vorrichtung nach einer der Ausführungsformen 1 bis 13, dadurch gekennzeichnet, dass die Vorrichtung (33) zum Einführen der Klammer (3) in eine Kartusche (34) oder einen Klammerspeicher (35) einen Schieber (40) zur Beaufschlagung der Klammer (3) an deren Klammerrücken (30) aufweist.
15. Vorrichtung nach Ausführungsform 14, dadurch gekennzeichnet, dass der Schieber (40) dem Drahtabschnitt (2) im Beaufschlagungsbereich formangepasst ist.
16. Vorrichtung nach Ausführungsform 14 oder 15, dadurch gekennzeichnet, dass der Schieber (40) im Beaufschlagungsbereich dünner ist als der zu beaufschlagende Drahtabschnitt (2).
17. Vorrichtung nach einer der Ausführungsformen 14 bis 16, dadurch gekennzeichnet, dass eine Antriebseinheit (46) für eine Linearverschiebung des Schiebers (40) vorgesehen ist.
18. Vorrichtung nach Ausführungsform 17, dadurch gekennzeichnet, dass die Antriebseinheit (46) einen Kraftsensor (48) sowie eine Steuereinheit zum Steuern des Schiebers (40) in Abhängigkeit der Sensor-Signale aufweist.
19. Vorrichtung nach Ausführungsform 17, dadurch gekennzeichnet, dass die Antriebseinheit (46) eine kraftgesteuerte Kupplungseinheit aufweist.
20. Vorrichtung nach einer der Ausführungsformen 1 bis 19, dadurch gekennzeichnet, dass die Vorrichtung (33) zum Einführen der Klammer (3) in eine Kartusche (34) oder einen Klammerspeicher (35) ein Gehäuse (36) mit einer Führung für den Schieber (40) sowie einer Einlegeöffnung (41) zum Einlegen jeweils einer Klammer (3) aufweist.
21. Vorrichtung nach einer der Ausführungsformen 1 bis 20, dadurch gekennzeichnet, dass eine Positioniervorrichtung (44) zum Positionieren eines Klammerspeichers (35) oder einer Kartusche (34) vor dem abgabeseitigen Ende der Vorrichtung (33) zum Einführen der Klammer (3) in eine Kartusche (34) oder einen Klammerspeicher (35) vorgesehen ist.
22. Vorrichtung nach einer der Ausführungsformen 1 bis 21, dadurch gekennzeichnet, dass die einzelnen Komponenten der Vorrichtung (1) an einer gemeinsamen Bearbeitungsstation angeordnet sind.
23. Verfahren zum Herstellen von Klammern (3), insbesondere chirurgischen Klammern, bei dem die freien Enden eines geraden Drahtabschnitts (2) zu Drahtschenkeln (29) umgebogen werden, **dadurch gekennzeichnet,** dass der Drahtabschnitt (2) von einem Draht (5) abgeschnitten und die freien Enden des Drahtabschnittes (2) zu Drahtschenkeln (29) umgebogen werden, dass der zur Klammer (3) gebogene Drahtabschnitt (2) in eine Kartusche (34) oder einen Klammerspeicher (35) eingeführt wird, und dass der Drahtabschnitt (2) vor dem Abschneiden gegriffen und während dem Abschneiden, dem Umbiegen , und bis zum Zuführen des zur Klammer gebogenen Drahtabschnitts an eine Vorrichtung zum Einführen der Klammer in eine Kartusche oder einen Klammerspeicher durchgängig gehalten wird.
24. Verfahren nach Ausführungsform 23, dadurch gekennzeichnet, dass der Draht (5) von einer Drahtrolle (7) abgewickelt wird.
25. Verfahren nach Ausführungsform 23 oder 24, dadurch gekennzeichnet, dass der Draht (5) vor dem Abschneiden ausgerichtet und/oder gereckt wird.
26. Verfahren nach einer der Ausführungsformen 23 bis 25, dadurch gekennzeichnet, dass der abgeschnittene Drahtabschnitt (2) mit seinem den Klammerrücken (30) der fertigen Klammer (3) bildenden mittleren Bereich an einen Amboss (23) angelegt und der Amboss (23) zwischen zwei druckbeaufschlagten Andrückrollen (25) hindurchbewegt wird und dadurch die über den Amboss (23) überstehenden Drahtbereiche umgebogen werden.
27. Verfahren nach einer der Ausführungsformen 23 bis 26, dadurch gekennzeichnet, dass die umgebogenen Drahtschenkel (29) aus der Klammer-Ebene heraus umgebogen und gegeneinander geschert werden.
28. Verfahren nach einer der Ausführungsformen 23 bis 27, dadurch gekennzeichnet, dass der Drahtabschnitt (2) vor und/oder nach dem Biegen bearbeitet wird.

## Patentansprüche

1. Vorrichtung (1) zum Herstellen von aus Drahtabschnitten (2) gebogenen Klammern (3), insbesondere chirurgischen Klammern, mit einer Biege-Einrichtung (22) zum Biegen eines Drahtabschnitts (2) und mit einer Zuführeinrichtung (4) für einen Draht (5) mit einer Schneideeinrichtung (6) zum Abtrennen eines Drahtabschnitts (2) von dem zugeführten Draht (5), **dadurch gekennzeichnet, dass** die Schneideeinrichtung (6) zwei Schneide-Stempel (11) zum Schneiden des Drahtes (5) aufweist, und dass eine Haltevorrichtung (14) zum Greifen und Halten des Drahtabschnittes (2) zwischen den beiden Schneide-Stempeln (11) vor dem Abtrennen des Drahtabschnittes (2) und zum Positionieren des abgetrennten Drahtabschnittes (2) an einer Biege-Einrichtung (22) sowie zum Zuführen des zur Klammer (3) gebogenen Drahtabschnitts (2) an eine Vorrichtung (33) zum Einführen der Klammer (3) in eine Kartusche (34) oder einen Klammerspeicher (35) vorgesehen ist.

2. Vorrichtung nach Anspruch 1, **dadurch gekennzeichnet, dass** die Haltevorrichtung (14) zangenartig ausgebildet ist und einen gegen eine Rückstellkraft aus einer Halteposition in eine Offenstellung bringbaren Haltebereich aufweist.

3. Vorrichtung nach einem der Ansprüche 1 bis 2, **dadurch gekennzeichnet, dass** die Biege-Einrichtung (22) einen Amboss (31) aufweist, der Anschlagflächen (32) für die umgebogenen Drahtschenkel (29) und diesen zugeordnete Andrückrollen (25) zum Scheren der Drahtschenkel (29) gegeneinander aufweist.

4. Vorrichtung nach Anspruch 3, **dadurch gekennzeichnet, dass** die Haltevorrichtung (14) mit dem Amboss (23, 31) der Biege-Einrichtung (22) lösbar verbindbar ist.

5. Vorrichtung nach einem der Ansprüche 1 bis 4, **dadurch gekennzeichnet, dass** die Zuführeinrichtung (4) zwei in Draht-Vorschubrichtung linearverschiebbare Klemmelemente (10) aufweist.

6. Vorrichtung nach einem der Ansprüche 1 bis 5, **dadurch gekennzeichnet, dass** die Schneide-Stempel (11) jeweils in einer Stempel-Führung (12) gelagert sind, die um eine in Draht-Längsrichtung orientierte Achse verschwenkbar und/oder bezüglich der Draht-Längsrichtung um 180° gedreht anordenbar ist.

7. Vorrichtung nach einem der Ansprüche 1 bis 6, **dadurch gekennzeichnet, dass** eine Kontrollvorrichtung für den gebogenen Drahtabschnitt (2) vorgesehen ist.

8. Vorrichtung nach einem der Ansprüche 1 bis 7, **dadurch gekennzeichnet, dass** die Vorrichtung (33) zum Einführen der Klammer (3) in eine Kartusche (34) oder einen Klammerspeicher (35) einen Schieber (40) zur Beaufschlagung der Klammer (3) an deren Klammerrücken (30) aufweist.

9. Vorrichtung nach Anspruch 8, **dadurch gekennzeichnet, dass** eine Antriebseinheit (46) für eine Linearverschiebung des Schiebers (40) vorgesehen ist.

10. Vorrichtung nach Anspruch 9, **dadurch gekennzeichnet, dass** die Antriebseinheit (46) einen Kraftsensor (48) sowie eine Steuereinheit zum Steuern des Schiebers (40) in Abhängigkeit der Sensor-Signale aufweist.

11. Vorrichtung nach Anspruch 9, **dadurch gekennzeichnet, dass** die Antriebseinheit (46) eine kraftgesteuerte Kupplungseinheit aufweist.

12. Vorrichtung nach einem der Ansprüche 1 bis 11, **dadurch gekennzeichnet, dass** die Vorrichtung (33) zum Einführen der Klammer (3) in eine Kartusche (34) oder einen Klammerspeicher (35) ein Gehäuse (36) mit einer Führung für den Schieber (40) sowie einer Einlegeöffnung (41) zum Einlegen jeweils einer Klammer (3) aufweist.

13. Vorrichtung nach einem der Ansprüche 1 bis 12, **dadurch gekennzeichnet, dass** eine Positioniervorrichtung (44) zum Positionieren eines Klammerspeichers (35) oder einer Kartusche (34) vor dem abgabeseitigen Ende der Vorrichtung (33) zum Einführen der Klammer (3) in eine Kartusche (34) oder einen Klammerspeicher (35) vorgesehen ist.

14. Verfahren zum Herstellen von Klammern (3), insbesondere chirurgischen Klammern, bei dem die freien Enden eines geraden Drahtabschnitts (2) zu Drahtschenkeln (29) umgebogen werden, **dadurch gekennzeichnet, dass** der Drahtabschnitt (2) von einem Draht (5) abgeschnitten und die freien Enden des Drahtabschnittes (2) zu Drahtschenkeln (29) umgebogen werden, dass der zur Klammer (3) gebogene Drahtabschnitt (2) in eine Kartusche (34) oder einen Klammerspeicher (35) eingeführt wird, und dass der Drahtabschnitt (2) vor dem Abschneiden gegriffen und während dem Abschneiden, dem Umbiegen, und bis zum Zuführen des zur Klammer (3) gebogenen Drahtabschnitts (2) an eine Vorrichtung (33) zum Einführen der Klammer (3) in eine Kartusche (34) oder einen Klammerspeicher (35) durchgängig gehalten wird.

15. Verfahren nach Anspruch 14, **dadurch gekennzeichnet, dass** die umgebogenen Drahtschenkel (29) aus der Klammer-Ebene heraus umgebogen und gegeneinander geschert werden.

## Claims

1. A device (1) for producing clamps (3) bent from wire sections (2), in particular surgical clamps, said device having a bending device (22) for bending a wire section (2), and a feed device (4) for a wire (5) is provided with a cutting device (6) for severing the wire section (2) from the supplied wire (5), **characterized in that** the cutting device (6) comprises two cutting punches (11) for cutting the wire (5), and a holding device (14) is provided for gripping and holding the wire section (2) between the two cutting punches (11) prior to severing the wire section (2) and for positioning the severed wire section (2) on a bending device (22) as well as for supplying the wire section bent to form the clamp (3) to a device (33) for introducing the clamp (3) into a cartridge (34) or a clamp storage (35).

2. The device as claimed in claim 1, **characterized in that** the holding device (14) is realized in a tongs-like manner and comprises a holding region which can be brought against a resetting force from a holding position into an open position.

3. The device as claimed in one of claims 1 to 2, **characterized in that** the bending device (22) comprises an anvil (31) which comprises stop surfaces (32) for bent wire legs (29) and pressing rollers (25) which are assigned to said wire legs for shearing the wire legs (29) against one another.

4. The device as claimed in claim 3, **characterized in that** the holding device (14) is releasably connectable to the anvil (23, 31) of the bending device (22).

5. The device as claimed in one of claims 1 to 4, **characterized in that** the feed device (4) comprises two clamping elements (10) which are linearly displaceable in an advancing direction of the wire.

6. The device as claimed in one of claims 1 to 5, **characterized in that** the cutting punches (11) are mounted in each case in a punch guide (12) which is pivotable about an axis oriented in a longitudinal direction of the wire and/or can be arranged rotated by 180° with reference to the longitudinal direction of the wire.

7. The device as claimed in one of claims 1 to 6, **characterized in that** a control device is provided for the bent wire section (2).

8. The device as claimed in one of claims 1 to 7, **characterized in that** the device (33) for introducing the clamp (3) into a cartridge (34) or a clamp storage (35) comprises a slide (40) for acting upon the clamps (3) on the clamp rear (30) thereof.

9. The device as claimed in claim 8, **characterized in that** a drive unit (46) is provided for a linear displacement of the slide (40).

10. The device as claimed in claim 9, **characterized in that** the drive unit (46) comprises a force sensor (48) and a control unit for controlling the slide (40) in dependence of the sensor signals.

11. The device as claimed in claim 9, **characterized in that** the drive unit (46) comprises a force-controlled coupling unit.

12. The device as claimed in one of claims 1 to 11, **characterized in that** the device (33) for introducing the clamp (3) into the cartridge (34) or the clamp storage (35) comprises a housing (36) with a guide for the slide (40) and an insertion opening (41) for inserting in each case one clamp (3).

13. The device as claimed in one of claims 1 to 12, **characterized in that** a positioning device (44) is provided for positioning the clamp storage (35) or the cartridge (34) in front of the output-side end of the device (33) for introducing the clamp (3) into the cartridge (34) or the clamp storage (35).

14. A method for producing clamps (3), in particular surgical clamps, where the free ends of a straight wire section (2) are bent to form wire legs (29), **characterized in that** the wire section (2) is severed from a wire (5) and the free ends of the wire section (2) are bent to form wire legs (29), **in that** the wire section (2) that is bent to form a clamp (3) is introduced into a cartridge (34) or a clamp storage (35), and **in that** the wire section (2) is gripped prior to severing and is held constantly during the severing, the bending and until the feeding of the wire section (2) bent to from a clamp (3) to a device (33) for introducing the clamp (3) into the cartridge (34) or the clamp storage (35).

15. The method as claimed in claim 14, **characterized in that** the bent wire legs (29) are bent out of the clamp plane and are sheared against one another.

## Revendications

1. Dispositif (1) pour fabriquer des agrafes (3) pliées à partir de segments de fil métallique (2), en particulier des agrafes chirurgicales, comprenant un dispositif de pliage (22) pour plier un segment de fil métallique (2) et un dispositif d'amenée (4) d'un fil métallique (5) avec un dispositif de coupe (6) pour séparer un segment de fil métallique (2) du fil métallique (5) amené, **caractérisé en ce que** le dispositif de coupe (6) présente deux poinçons de coupe (11) pour couper le fil métallique (5), et **en ce qu'**un dispositif de maintien (14) est prévu pour saisir et maintenir le segment de fil métallique (2) entre les deux poinçons de coupe (11) avant la séparation du segment de fil métallique (2) et pour positionner le segment de fil métallique (2) séparé sur un dispositif de pliage (22) ainsi que pour amener le segment de fil métallique (2) plié en une agrafe (3) à un dispositif (33) pour introduire l'agrafe (3) dans une cartouche (34) ou dans un magasin à agrafes (35).

2. Dispositif selon la revendication 1, **caractérisé en ce que** le dispositif de maintien (14) est réalisé à la manière d'une pince et présente une zone de maintien pouvant être amenée d'une position de maintien à une position ouverte en s'opposant à une force de rappel.

3. Dispositif selon l'une des revendications 1 ou 2, **caractérisé en ce que** le dispositif de pliage (22) présente une enclume (31) qui présente des surfaces de butée (32) pour les branches de fil métallique (29) pliées et des galets presseurs (25) associés à celles-ci pour mettre les branches de fil métallique (29) en ciseau l'une par rapport à l'autre.

4. Dispositif selon la revendication 3, **caractérisé en ce que** le dispositif de maintien (14) peut être relié de manière amovible à l'enclume (23, 31) du dispositif de pliage (22).

5. Dispositif selon l'une des revendications 1 à 4, **caractérisé en ce que** le dispositif d'amenée (4) présente deux éléments de serrage (10) déplaçables linéairement dans la direction d'avance du fil métallique.

6. Dispositif selon l'une des revendications 1 à 5, **caractérisé en ce que** les poinçons de coupe (11) sont montés chacun dans un guide de poinçon (12) qui peut pivoter autour d'un axe orienté dans la direction longitudinale du fil métallique et/ou peut être disposé tourné de 180° par rapport à la direction longitudinale du fil métallique.

7. Dispositif selon l'une des revendications 1 à 6, **caractérisé en ce qu'**un dispositif de contrôle du segment de fil métallique (2) plié est prévu.

8. Dispositif selon l'une des revendications 1 à 7, **caractérisé en ce que** le dispositif (33) pour introduire l'agrafe (3) dans une cartouche (34) ou dans un magasin à agrafes (35) présente un poussoir (40) destiné à agir sur l'agrafe (3) au niveau de son dos d'agrafe (30).

9. Dispositif selon la revendication 8, **caractérisé en ce qu'**une unité d'entraînement (46) pour un déplacement linéaire du poussoir (40) est prévue.

10. Dispositif selon la revendication 9, **caractérisé en ce que** l'unité d'entraînement (46) présente un capteur de force (48) ainsi qu'une unité de commande pour commander le poussoir (40) en fonction des signaux du capteur.

11. Dispositif selon la revendication 9, **caractérisé en ce que** l'unité d'entraînement (46) présente une unité d'accouplement commandée par force.

12. Dispositif selon l'une des revendications 1 à 11, **caractérisé en ce que** le dispositif (33) pour introduire l'agrafe (3) dans une cartouche (34) ou dans un magasin à agrafes (35) présente un boîtier (36) avec un guide pour le poussoir (40) ainsi qu'une ouverture d'insertion (41) pour l'insertion d'une agrafe (3) à la fois.

13. Dispositif selon l'une des revendications 1 à 12, **caractérisé en ce qu'**un dispositif de positionnement (44) est prévu pour positionner un magasin à agrafes (35) ou une cartouche (34) devant l'extrémité côté sortie du dispositif (33) pour introduire l'agrafe (3) dans une cartouche (34) ou dans un magasin à agrafes (35).

14. Procédé pour fabriquer des agrafes (3), en particulier des agrafes chirurgicales, selon lequel les extrémités libres d'un segment de fil métallique (2) droit sont pliées en branches de fil métallique (29), **caractérisé en ce que** le segment de fil métallique (2) est coupé à partir d'un fil métallique (5) et les extrémités libres du segment de fil métallique (2) sont pliées pour former des branches de fil métallique (29), **en ce que** le segment de fil métallique (2) plié en une agrafe (3) est introduit dans une cartouche (34) ou dans un magasin à agrafes (35), et **en ce que** le segment de fil métallique (2) est saisi avant la coupe et maintenu sans interruption pendant la coupe, le pliage et jusqu'à l'amenée du segment de fil métallique (2) plié en une agrafe (3) à un dispositif (33) pour introduire l'agrafe (3) dans une cartouche (34) ou un magasin à agrafes (35).

15. Procédé selon la revendication 14, **caractérisé en ce que** les branches de fil métallique (29) pliées sont pliées hors du plan de l'agrafe et mises en ciseau l'une par rapport à l'autre.
